# EUROPEAN PATENT SPECIFICATION

(11) **EP 4 065 069 B1**
(45) Date of publication and mention of the grant of the patent: **11.03.2026**
(21) Application number: 20828809.2
(22) Date of filing: 01.12.2020
(51) Int. Cl.: A61K 8/04, A61K 8/34, A61K 8/368, A61K 8/37, A61K 8/41, A61Q 5/00, A61Q 5/12, A61K 8/42, A61K 8/92

(54) **HAIR CONDITIONER COMPOSITIONS CONTAINING BEHENAMIDOPROPYL DIMETHYLAMINE**
HAARKONDITIONIERENDE ZUBEREITUNGEN ENTHALTEND BEHENAMIDOPROPYLDIMETHYLAMIN
COMPOSITIONS CAPILLAIRES CONDITIONNANTES CONTENANT LA DIMÉTHYLAMINE DE BEHENAMIDOPROPYL

(30) Priority: 01.12.2019 US 201962942208 P
(43) Date of publication of application: 05.10.2022
(73) Proprietor: The Procter & Gamble Company, Cincinnati, OH 45202 (US)
(72) Inventor: ZHAO, Jean, Jianqun, Cincinnati, Ohio 45202 (US); BALLHAUS, Lauren, Elizabeth, Cincinnati, Ohio 45202 (US); FOCHT, Heather, Lynn, Cincinnati, Ohio 45202 (US); KAUFMAN, David, Joseph, Cincinnati, Ohio 45202 (US); MCQUEEN, Christina, Marie, Cincinnati, Ohio 45202 (US); MULLIGAN, Deborah, Cincinnati, Ohio 45202 (US); WITTE, Lina, Aurora, Cincinnati, Ohio 45202 (US)
(74) Representative: P&G Patent Germany
(86) International application number: PCT/US2020/062654
(87) International publication number: WO 2021/113209

(56) References cited:
- EP-A1- 1 808 157
- DE-A1- 102010 026 747
- US-A1- 2006 024 256
- US-A1- 2007 237 736
- US-A1- 2010 150 858
- US-A1- 2015 250 701

## Description

### FIELD OF THE INVENTION

The present invention relates to hair conditioner compositions, more particularly to hair conditioner compositions comprising behenamidopropyl dimethylamine cationic surfactant.

### BACKGROUND OF THE INVENTION

A variety of approaches have been developed to condition the hair. These approaches range from post-shampoo application of hair conditioners such as leave-on and rinse-off products, to hair conditioning shampoos which attempt to both clean and condition the hair from a single product.

Although some consumers prefer the ease and convenience of a shampoo which includes conditioners, a substantial proportion of consumers prefer the more conventional conditioner formulations which are applied to the hair as a separate step from shampooing, usually after shampooing. Conditioning formulations can be in the form of rinse-off products or leave-on products, and can be in the form of an emulsion, cream, gel, spray, and mousse. Such consumers who prefer the conventional conditioner formulations value the relatively higher conditioning effect, or convenience of changing the amount of conditioning depending on the condition of hair or amount of hair.

Some consumers want a reduction or elimination of certain ingredients, including certain surfactants and preservatives, in hair care products. Some consumers want the ingredients in their hair care products to be EWG VERIFIED^{™}, free of any of the ingredients that Whole Foods^{®} lists as unacceptable for body care and be categorized as "risk-free" (green dot) by the Yuka^{®} Application.

The US patent application US-A-2010/150858 discloses a hair conditioning composition comprising by weight, from 0.2% to 10% of behenamidopropyldimethylamine as cationic surfactant, from 1% to 15% of fatty alcohols as high melting point fatty compound and an aqueous carrier. The US patent application US-A-2007/237736 discloses hair conditioning formulations comprising stearamidopropyl dimethylamine, behenamidopropyl dimethylamine and fatty alcohols such as Stearyl alcohol and Cetyl alcohol.

However, modifying the conditioner can negatively impact the product. For example, modifying the cationic surfactant can decrease conditioning performance and modifying the preservation system can have a negative impact on microbiological safety requirements.

Therefore, there is a need for a conditioner composition with an effective surfactant and preservation system that that is EWG VERIFIED^{™}, does not contain any of the ingredients that Whole Foods^{®} Market lists as unacceptable, and is categorized as "risk-free" by Yuka^{®} Application.

### SUMMARY OF THE INVENTION

A hair conditioner composition as required in claim 1, comprises (a) an aqueous carrier; (b) from 2.75 wt% to 6 wt% of a behenamidopropyl dimethylamine; (c) from about 3 wt% to about 8 wt% of a fatty alcohol; (d) less than 1.5 wt% of a salt selected from the group consisting of sodium benzoate, sodium salicylate, sodium chloride, sodium carbonate, sodium borate, sodium acetate, sodium citrate, potassium benzoate, potassium acetate, calcium gluconate, calcium chloride, potassium sorbate, and combinations thereof; wherein the molar ratio of behenamidopropyl dimethylamine to fatty alcohol is from about from 9:50 to 2:3; wherein the composition comprises a uniform gel network; wherein the composition comprises d-spacing of less than 32 nm, as measured according to the d-spacing (Lβ-basal spacing) of Lamella Gel Network Test Method.

### BRIEF DESCRIPTION OF THE DRAWINGS

While the specification concludes with claims particularly pointing out and distinctly claiming the subject matter of the present invention, it is believed that the invention can be more readily understood from the following description taken in connection with the accompanying drawings, in which:
FIG. 1A shows the chemical structure for behenamidopropyl dimethylamine (BAPDMA);
FIG. 1B shows the chemical structure of BAPDMA after it has been activated with ℓ-glutamic acid;
FIG. 2 shows the melt transition behavior of the gel network, as measured by the Differential Scanning Calorimetry (DSC) Test Method, of Examples 1-5 and Comparative Examples A and B;
FIG. 3 is a photograph of the conditioner composition of Comparative Example J, which contains 1 wt% sodium benzoate and has a clumpy and grainy appearance;
FIG. 4 is a photograph of the conditioner composition of Comparative Example K, which contains 2 wt% sodium benzoate and has a thin and grainy appearance;
FIG. 5 is a photograph of the conditioner composition of Example A, which contains 0.20 wt% sodium benzoate and 0.40 wt% caprylyl glycol.

### DETAILED DESCRIPTION OF THE INVENTION

While the specification concludes with claims particularly pointing out and distinctly claiming the invention, it is believed that the present invention will be better understood from the following description.

Hair conditioners are used to improve the feel, appearance, and manageability of the hair. Hair conditioning compositions generally include cationic surfactant(s), high melting point fatty compound(s) having a melting point of greater than 25°C and in some examples from 40 to 85°C, and an aqueous carrier. The ingredients in current hair conditioners, including the cationic surfactant and preservation system, are generally recognized as safe and effective.

However, there is growing demand from some consumers for a conditioner product and/or a preservation system that meets at least one, two, or all three of the following standards:
- EWG VERIFIED^{™} (according to the criteria, as of November 25, 2019), which includes meeting the Environmental Working Group's (EWG) criteria including avoiding EWG's ingredients of concern, having fully transparent labeling, and using good manufacturing practices, in addition to other criteria described in EWG's Licensing Criteria: Personal Care Products (2019).
- Does not contain any of ingredients that Whole Foods^{®} lists as unacceptable lists as unacceptable in its *Premium Body Care Unacceptable Ingredients* (July 2018)
- Categorized as "risk-free" (green dot) by the Yuka^{®} Application (March 2019)

However, replacing traditional cationic surfactants, such as behentrimonium chloride (BTMAC), which is restricted by the Environmental Working Group (EWG) for use in the cosmetic products as of November 25, 2019 and/or stearamidopropyl dimethylamine (SAPDMA), which is an unacceptable ingredient listed on the Whole Foods^{®} *Premium Body Care Unacceptable Ingredients* (July 2018), and preservatives with ingredients that meet the standards, listed above, while maintaining product performance and antimicrobial effectiveness can be challenging.

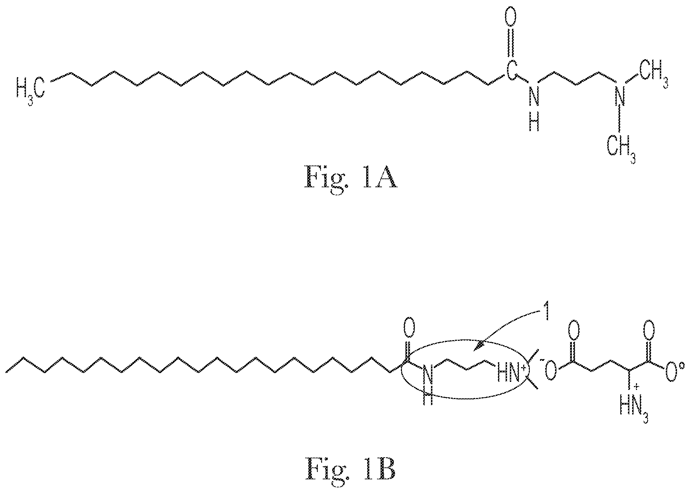

Behenamidopropyl dimethylamine (BAPDMA) was identified as a cationic surfactant that can meet the standards, above. However, it can be hard to formulate effective conditioner compositions that contain BAPDMA because BAPDMA is a C₂₂ amidoamine with the structure shown in FIG. 1A. BAPDMA needs to be activated with an acid, like ℓ-glutamic acid, to become cationic. However, after the cationic surfactant is formed, as shown in the structure in FIG. 1B, it has a large head group 1. This large head group tends to affect the surfactant and fatty alcohol(s) packing during the melt stage when the gel network is formed. This makes the d-spacing in the gel network in the conditioner composition larger, which generally leads to a conditioner with poor wet conditioning performance.

It was surprisingly found that the d-spacing in the inventive conditioner compositions (see Table 3 to Table 7, below) is less than 30 nm. It is believed that conditioners with d-spacing of less than 30 nm can provide good conditioning, clean feel, volume, and consumer preferred shear stress.

While not willing to be bound by theory, it is believed that the molar ratio of BAPDMA to fatty alcohol(s) and the addition of salts including sodium benzoate, can result in a conditioner composition with d-spacing that is less than 30 nm that provides good conditioning performance including a good slippery feel and wet detangling.

Table 1, below, compares the d-spacing of four commercially available conditioner compositions to Ex. 1 to Ex. 28 in Table 3 to Table 7. It was found that the d-spacing of Ex. 1 to 28 was similar to Herbal Essences^{®} Honey & Vitamin B Conditioner, which contains BTMAC and the d-spacing was lower than the other examples, including John Frieda^{®} Sheer Blond Brightening Conditioner, which contains the same cationic polymer (BAPDMA) as Ex. 1-28.

**Table 1: d-Spacing of Current Products vs. Examples 1-28**

| | Herbal Essences^{®} Honey & Vitamin B Conditioner¹ | Herbal Essences^{®} Cucumber & Green Tea Conditioner² | Herbal Essences^{®} Refresh Blue Ginger³ | John Frieda^{®} Sheer Blond Brightening Conditioner⁴ | Ex. 1 to Ex. 28 (Table 3 to Table 7) |
|---|---|---|---|---|---|
| Primary Cationic Surfactant | BTMAC | SAPDMA | SAPDMA | BAPDMA | BAPDMA |
| Gel network d-spacing (nm) | 24.2 | 32.6 | 34 | 35.1 | 20.8 to 26.8 |

| | | | | | |
|---|---|---|---|---|---|
| 1. Lot # 83565395LF, purchased at Target^{®}, Mason, Ohio 2019 2. Lot # 82325395LK, purchased at Target^{®}, Mason, Ohio 2019 3. Lot # 82165395LC, purchased at Target^{®}, Mason, Ohio 2019 4. Lot # X0J11438 B, purchased at Target^{®}, Mason, Ohio 2019 | | | | | |

Furthermore, the conditioners in Ex. 1 to Ex. 28 (Table 3 to Table 7), had a uniform gel network, as indicated by the single peak when the Differential Scanning Calorimetry Test Method was performed.

Also, conditioner compositions that provide good wet conditioning may weigh down hair causing a loss of dry hair volume and/or may cause the hair to feel dirty and/or oily quickly, which can result in consumers washing their hair more frequently. It was found that the hair conditioner compositions of Ex. 1 to Ex. 35 may not only provide good hair conditioning but may also give good hair volume and/or provide a long-lasting clean feel, allowing less washing frequency.

Furthermore, Table 9 and Table 10, below, include examples that have a sodium benzoate preservative, which meets the standards. However, if the conditioner composition had a smooth and creamy consistency, then the level of sodium benzoate was too low to effectively inhibit the growth of microbes. When the level of sodium benzoate was increased, the conditioner composition was too thin to easily apply with a user's hands, which can significantly impact product performance and the usage experience. As shown in Table 11 and described in accompanying text, a preservation system with sodium benzoate and a glycol, such as caprylyl glycol, or glyceryl esters, such as glyceryl caprylate/caprate and glyceryl caprylate (and) glyceryl undecylenate can be effective if the proper levels of each ingredient are added.

It was found that a preservation system that contains sodium benzoate and a second composition selected from the group consisting of glycols, glyceryl esters, and combinations thereof contains all of the ingredients that have a EWG rating score of equal to or less than 3, can be EWG VERIFIED^{™}, may not contain any of the ingredients that Whole Foods^{®} Market lists as unacceptable, and can categorized as "risk-free" by the Yuka^{®} Application, can maintain antimicrobial effectiveness, and can provide good conditioning performance.

The second composition can contain a glycol and/or a glyceryl ester. Glycols and glyceryl esters both have two -OH groups on the molecule. Non-limiting examples of glycols can include butylene glycol, pentylene glycol, hexylene glycol, 1.2-hexanediol, caprylyl glycol, decylene glycol (1,2-decanediol) and mixtures thereof. In one example, the glycol can be carpylyl glycol. Non-limiting examples of glycerol esters can include glyceryl caprylate, glyceryl caprate, glyceryl undecylenate and mixtures thereof.

In some examples, the cationic surfactant, the preservation system and/or the conditioner can also meet the COSMOS-standard (January 1, 2019). The conditioner compositions containing this preservation system can have a uniform, smooth, creamy appearance and have an effective preservation system where the level of microbes (both bacteria and fungi) is undetectable (>99.99% reduction) as determined by the Bacterial and Fungal Microbial Susceptibility Test Methods, as described herein.

The conditioner composition and/or preservation system can be free of or substantially free of certain preservatives, in particular preservatives that do not meet one or more of the requirements, isothiazolinones including 5-chloro-2-methyl-4-isothiazolin-3-one and 2-methyl-4-isothiazolin-3-one (commercially available as Kathon^{™} CG from Dow^{®}), benzyl alcohol, phenoxyethanol, cyclohexylglycerin, and/or parabens. The conditioner composition can be free of or substantially free of ethylenediaminetetraacetic acid (EDTA) and salts thereof.

In addition to meeting the standards for a cationic surfactant and preservation system, some consumers prefer a conditioner composition that is free of or substantially free of the following: silicone, propellants, phthalates, parabens, isothiazolinones (e.g. Kathon^{™}), phenoxyethanols, dyes, sulfates, and/or formaldehyde donors. The conditioner composition can also be vegan.

The conditioner composition can be free of or substantially free of behentrimonium chloride, cetrimonium chloride, and/or stearamidopropyl dimethylamine.

The conditioner composition can contain less than 6.75 wt% BAPDMA, alternatively less than about 6.50 wt% BAPDMA, alternatively less than about 6.25 wt% BAPDMA. The conditioner composition can contain from about 2 wt% to about 6.7 wt% BAPDMA, alternatively from about 2.2 wt% to about 6.5 wt%, alternatively from about 2.5 wt% to about 6.25 wt% BAPDMA, alternatively from about 2.75 wt% to about 6 wt%.

The conditioner composition can contain a fatty alcohol selected from the group consisting of stearyl alcohol, brassical alcohol, and cetyl alcohol. The conditioner composition can contain less than 8 wt% fatty alcohol, alternatively less than 7.5 wt%, alternatively less than 7 wt%. The conditioner composition can contain from about 2.5 wt% to about 9 wt% fatty alcohol, alternatively from about 3 wt% to about 8 wt%, alternatively from about 3.25 wt% to about 7.5 wt%, alternatively from about 3.5 wt% to about 7 wt%, alternatively from about 4 wt% to about 6.7 wt%.

The conditioner composition can have a total gel network (GN) content (BAPDMA + fatty alcohol(s) (FAOH)) of from about 0.1 to about 0.6 molar, alternatively from about 0.2 to about 0.5 molar, alternatively from about 0.3 to about 0.4 molar.

The conditioner composition can have a molar ratio of stearyl alcohol (C18 fatty alcohol) to total FAOH of from about 1:10 to about 1:1, alternatively from about 1:5 to about 9:10, alternatively from about 3:10 to about 4:5, and alternatively from about 2:5 to about 7:10.

The conditioner composition can have a molar ratio of acid to BAPDMA ratio of from about 1:2 to about 7:4, alternatively from about 3:5 to about 1:2, alternatively from about 7:10 to about 5:4, and alternatively from about 4:5 to about 1.3:1.

The conditioner composition can have a molar ratio of BAPDMA to FAOH from about 3:25 to about 9:10, from about 7:50 to about 4:5, from about 3:20 to about 3:4, from about 17: 100 to about 7:10, and alternatively from about 9:50 to about 2:3.

The conditioner composition can have d-spacing of less than 32 nm, alternatively less than 30 nm, and alternatively less than 28 nm. The conditioner composition can have d-spacing from about 10 nm to about 30 nm, alternatively from about 15 nm to about 30 nm, alternatively from about 17 nm to about 29 nm, alternatively from about 18 nm to about 28 nm, and alternatively from about 20 nm to about 27 nm. The d-spacing determined by the the d-spacing (Lβ-basal spacing) of Lamella Gel Network Test Method, described herein.

The conditioner composition can contain from about 0.2 wt% to about 1.5 wt% preservation system, alternatively from about 0.3 wt% to about 1.25 wt% preservation system, alternatively from about 0.4 wt% to about 1 wt% preservation system, alternatively from 0.5 wt% to about 0.8 wt% preservation system, and alternatively from about 0.6 wt% to about 0.8 wt% preservation system.

The conditioner composition can contain from about 0.05 wt% to about 0.8 wt% sodium benzoate, alternatively 0.1 wt% to about 0.5 wt% sodium benzoate, alternatively from about 0.2 wt% to about 0.4 wt% sodium benzoate. The conditioner composition can contain sodium benzoate and can contain less than 2% sodium benzoate, alternatively less than 1.5% sodium benzoate, alternatively less than 1% sodium benzoate, alternatively less than 0.8% sodium benzoate, alternatively less than 0.6 wt% sodium benzoate, and alternatively less than 0.5% sodium benzoate.

The preservation system can contain from about 20% to about 50% sodium benzoate, by weight of the preservation system, alternatively from about 25% to about 50% sodium benzoate, by weight of the preservation system, from about 30% to about 50% sodium benzoate, by weight of the preservation system, and from about 30% to about 40% sodium benzoate, by weight of the preservation system.

The conditioner composition can contain from about 0.3 wt% to about 1.5 wt% of a second composition, alternatively from about 0.32 wt% to about 1 wt%, alternatively from about 0.33 wt% to about 0.8 wt%, alternatively from about 0.34 wt% to about 0.6 wt%, alternatively from about 0.35 wt% to about 0.5 wt%, alternatively from about 0.37 wt% to about 0.45 wt%, and alternatively from about 0.38 wt% to about 0.43 wt%. If the conditioner composition contains too much glycol and/or glyceryl esters the gel network structure may be destroyed, and the conditioner will not have consumer acceptable rheology and/or performance.

The preservation system can contain from about 50% to about 80% of the second composition, by weight of the preservation system, alternatively from about 50% to about 75%, by weight of the preservation system, alternatively from about 50% to about 70%, by weight of the preservation system, and alternatively from about 50% to about 67%, by weight of the preservation system.

The weight ratio of sodium benzoate to the second composition can be from about 1:4 to about 1:1, alternatively from about 1:3 to about 1:1, alternatively from about 1:2 to about 1:1, and from about 1:1.7 to about 1:1.

The conditioner composition can have a shear stress from about 50 Pa to about 600 Pa, alternatively from about 75 Pa to about 575 Pa, alternatively from about 100 Pa to about 565 Pa, alternatively from about 105 Pa to about 550 Pa, alternatively from about 120 Pa, to about 500 Pa, and alternatively from about 125 Pa to about 450 Pa. The shear stress can be determined using the Shear Stress Test Method, described hereafter.

The conditioner composition can have a pH of less than 5. Alternatively, the conditioner composition can have a pH from about 2.5 to about 5, alternatively from about 3.5 to about 4.5. The pH can be determined using the pH Test Method, described hereafter.

As used herein, the articles including "a" and "an" when used in a claim, are understood to mean one or more of what is claimed or described.

As used herein, "comprising" means that other steps and other ingredients which do not affect the end result can be added. This term encompasses the terms "consisting of" and "consisting essentially of."

As used herein, the terms "include," "includes," and "including," are meant to be non-limiting and are understood to mean "comprise," "comprises," and "comprising," respectively.

As used herein, the term "free of" means that 0% of an ingredient was intentionally added to the conditioner composition, or the conditioner composition comprises 0% of an ingredient by total weight of the composition, thus no detectable amount of the stated ingredient.

The term "substantially free of" as used herein means less than 0.5%, less than 0.3%, less than 0.1%, less than 0.05%, less than 0.01%, or less than an immaterial amount of a stated ingredient by total weight of the composition.

As used herein, "mixtures" is meant to include a simple combination of materials and any compounds that may result from their combination.

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Unless otherwise noted, all component or composition levels are in reference to the active portion of that component or composition, and are exclusive of impurities, for example, residual solvents or by-products, which may be present in commercially available sources of such components or compositions.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

### CATIONIC SURFACTANT

The compositions of the present invention can comprise a cationic surfactant. The cationic surfactant can be included in the composition at a level of from about 0.1%, alternatively from about 0.5%, alternatively from about 0.8%, alternatively from about 1.0%, and to about 20%, alternatively to about 10%, alternatively to about 8.0%, alternatively to about 6.0% by weight of the composition, in view of providing the benefits of the present invention.

The surfactant can be water-insoluble. In the present invention, "water-insoluble surfactants" means that the surfactants have a solubility in water at 25°C of alternatively below 0.5g/100g (excluding 0.5g/100g) water, alternatively 0.3g/100g water or less.

Cationic surfactant can be one cationic surfactant or a mixture of two or more cationic surfactants. Alternatively, the cationic surfactant is selected from: a mono-long alkyl amine; a di-long alkyl quaternized ammonium salt; a mono-long alkyl cationic neutralized amino acid esters; a combination of a mono-long alkyl amine and a di-long alkyl quaternized ammonium salt; and a combination of a mono-long alkyl amine and a mono-long alkyl cationic neutralized amino acid esters.

In some examples, the conditioner composition can be substantially free of or free of cationic surfactants that have a quaternized ammonium salt.

### Mono-long alkyl amine

Mono-long alkyl amine can include those having one long alkyl chain of alternatively from 19 to 30 carbon atoms, alternatively from 19 to 24 carbon atoms, alternatively from 20 to 24 carbon atoms, alternatively from 20 to 22 alkyl group. Mono-long alkyl amines can include mono-long alkyl amidoamines. Primary, secondary, and tertiary fatty amines can be used.

Tertiary amido amines having an alkyl group of from about 19 to about 22 carbons. Exemplary tertiary amido amines include: behenamidopropyldimethylamine, behenamidopropyldiethylamine, behenamidoethyldiethylamine, behenamidoethyldimethylamine, brassicamidopropyldimethylamine, brassicamidopropyldiethylamine, brassicamidoethyldiethylamine, brassicamidoethyldimethylamine. Amines in the present invention are disclosed in U.S. Patent 4,275,055, Nachtigal, et al.

In some examples, the conditioner composition can be substantially free of or free of stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, arachidamidopropyldimethylamine, arachidamidopropyldiethylamine, arachidamidoethyldiethylamine, arachidamidoethyldimethylamine, and/or diethylaminoethylstearamide.

These amines are used in combination with acids such as ℓ-glutamic acid, lactic acid, hydrochloric acid, malic acid, succinic acid, acetic acid, fumaric acid, tartaric acid, citric acid, ℓ-glutamic hydrochloride, maleic acid, and mixtures thereof; alternatively lactic acid, citric acid, at a molar ratio of the amine to the acid of from about 1:0.3 to about 1:2, alternatively from about 1:0.4 to about 1:1. The conditioner composition can contain from about 0.25 wt% to about 6 wt% acid, alternatively from about 0.4 wt% to about 5 wt% acid, from about 0.5 wt% to about 4 wt% acid, and alternatively from about 0.6 wt% to about 3 wt% acid.

**In** some examples, the conditioner composition can be free of mono long alkyl quaternized ammonium salts.

### Di-long alkyl quaternized ammonium salts

When used, di-long alkyl quaternized ammonium salts are alternatively combined with a mono-long alkyl quaternized ammonium salt and/or mono-long alkyl amine salt, at the weight ratio of from 1:1 to 1:5, alternatively from 1:1.2 to 1:5, alternatively from 1:1.5 to 1:4, in view of stability in rheology and conditioning benefits.

Di-long alkyl quaternized ammonium salts can have two long alkyl chains of from 12 to 30 carbon atoms, alternatively from 16 to 24 carbon atoms, alternatively from 18 to 22 carbon atoms. Such di-long alkyl quaternized ammonium salts can have the formula (I): 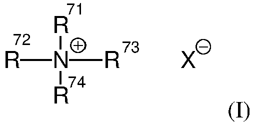 wherein two of R⁷¹, R⁷², R⁷³ and R⁷⁴ are selected from an aliphatic group of from 12 to 30 carbon atoms, alternatively from 16 to 24 carbon atoms, alternatively from 18 to 22 carbon atoms or an aromatic, alkoxy, polyoxyalkylene, alkylamido, hydroxyalkyl, aryl or alkylaryl group having up to about 30 carbon atoms; the remainder of R⁷¹, R⁷², R⁷³ and R⁷⁴ are independently selected from an aliphatic group of from 1 to about 8 carbon atoms, alternatively from 1 to 3 carbon atoms or an aromatic, alkoxy, polyoxyalkylene, alkylamido, hydroxyalkyl, aryl or alkylaryl group having up to about 8 carbon atoms; and X⁻ is a salt-forming anion selected from the group consisting of halides such as chloride and bromide, C1-C4 alkyl sulfate such as methosulfate and ethosulfate, and mixtures thereof. The aliphatic groups can contain, in addition to carbon and hydrogen atoms, ether linkages, and other groups such as amino groups. The longer chain aliphatic groups, e.g., those of about 16 carbons, or higher, can be saturated or unsaturated. Alternatively, two of R⁷¹, R⁷², R⁷³ and R⁷⁴ are selected from an alkyl group of from 12 to 30 carbon atoms, alternatively from 16 to 24 carbon atoms, alternatively from 18 to 22 carbon atoms; and the remainder of R⁷¹, R⁷², R⁷³ and R⁷⁴ are independently selected from CH₃, C₂H₅, C₂H₄OH, CH₂C₆H₅, and mixtures thereof.

Di-long alkyl cationic surfactants can include, for example, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride.

### HIGH MELTING POINT FATTY COMPOUND

The composition of the present invention comprises a high melting point fatty compound. The high melting point fatty compound can be included in the composition at a level of from about 1.0%, alternatively from about 1.5%, alternatively from about 2.0%, alternatively from about 2.5%, even alternatively from about 3%, and to about 30%, alternatively to about 15%, alternatively to about 8.0%, alternatively to about 7% by weight of the composition, in view of providing the benefits of the present invention.

The high melting point fatty compound can have a melting point of 25°C or higher, alternatively 40°C or higher, alternatively 45°C or higher, alternatively 47°C or higher, alternatively 49°C or higher, in view of stability of the emulsion especially the gel network. Alternatively, such melting point is up to about 90°C, alternatively up to about 80°C, alternatively up to about 75°C, even alternatively up to about 71°C, in view of easier manufacturing and easier emulsification. In the present invention, the high melting point fatty compound can be used as a single compound or as a blend or mixture of at least two high melting point fatty compounds. When used as such blend or mixture, the above melting point means the melting point of the blend or mixture.

The high melting point fatty compound can be selected from the group consisting of fatty alcohols, fatty acids, and mixtures thereof. Further, it is understood by the artisan that, depending on the number and position of double bonds, and length and position of the branches, certain compounds having certain required carbon atoms may have a melting point of less than the above preferred in the present invention. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

Among a variety of high melting point fatty compounds, fatty alcohols are alternatively used in the composition of the present invention. The fatty alcohols can have from about 14 to about 30 carbon atoms, alternatively from about 16 to about 22 carbon atoms. These fatty alcohols are saturated and can be straight or branched chain alcohols.

Fatty alcohols can include, for example, cetyl alcohol (having a melting point of about 56°C), stearyl alcohol (having a melting point of about 58-59°C), behenyl alcohol (having a melting point of about 71°C), and mixtures thereof. These compounds are known to have the above melting point. However, they often have lower melting points when supplied, since such supplied products are often mixtures of fatty alcohols having alkyl chain length distribution in which the main alkyl chain is cetyl, stearyl, brassica or behenyl group.

The fatty alcohol can be a mixture of cetyl alcohol and stearyl alcohol.

Generally, in the mixture, the weight ratio of cetyl alcohol to stearyl alcohol is alternatively from about 1:9 to 9:1, alternatively from about 1:4 to about 4:1, alternatively from about 1:2.3 to about 1.5:1.

When using higher level of total cationic surfactant and high melting point fatty compounds, the mixture has the weight ratio of cetyl alcohol to stearyl alcohol of alternatively from about 1:1 to about 4:1, alternatively from about 1:1 to about 2:1, alternatively from about 1.2:1 to about 2:1, in view of avoiding to get too thick for spreadability. It may also provide more conditioning on damaged part of the hair.

### AQUEOUS CARRIER

The composition of the present invention can include an aqueous carrier. The level and species of the carrier can be selected according to the compatibility with other components, and other desired characteristic of the product.

The carrier can include water and water solutions of lower alkyl alcohols. The lower alkyl alcohols can be monohydric alcohols having 1 to 6 carbons, alternatively ethanol and isopropanol.

Alternatively, the aqueous carrier is substantially water. Deionized water is alternatively used. Water from natural sources including mineral cations can also be used, depending on the desired characteristic of the product. Generally, the compositions of the present invention comprise from about 40% to about 99%, alternatively from about 50% to about 95%, and alternatively from about 70% to about 93%, and alternatively from about 80% to about 92% water.

### GEL NETWORK

The gel network composition can be included in conditioner compositions to provide conditioning benefits, including improved wet feel of the hair after rinsing the conditioner. As used herein, the term "gel network" refers to a lamellar or vesicular solid crystalline phase which comprises at least one high melting point fatty compound, such as a fatty alcohol, as specified below, at least one surfactant, in particular a cationic surfactant, as specified below, and water or other suitable solvents. The lamellar or vesicular phase comprises bi-layers made up of a first layer comprising the high melting point fatty compound and the surfactant and alternating with a second layer comprising the water or other suitable solvent. Gel networks, generally, are further described by G. M. Eccleston, "Functions of Mixed Emulsifiers and Emulsifying Waxes in Dermatological Lotions and Creams", Colloids and Surfaces A: Physiochem. and Eng. Aspects 123-124 (1997) 169-182; and by G. M Eccleston, "The Microstructure of Semisolid Creams", Pharmacy International, Vol. 7, 63-70 (1986).

A gel network can be formed by the cationic surfactant, the high melting point fatty compound, and an aqueous carrier. The gel network is suitable for providing various conditioning benefits, such as slippery feel during the application to wet hair and softness and moisturized feel on dry hair.

Alternatively, when the gel network is formed, the cationic surfactant and the high melting point fatty compound are contained at a level such that the weight ratio of the cationic surfactant to the high melting point fatty compound is in the range of, alternatively from about 1:1 to about 1:10, alternatively from about 1:1.5 to about 1:7, alternatively from about 1:2 to about 1:6, in view of providing improved wet conditioning benefits.

Alternatively, especially when the gel network is formed, the composition of the present invention is substantially free of anionic surfactants, in view of stability of the gel network. In the present invention, "the composition being substantially free of anionic surfactants" means that: the composition is free of anionic surfactants; or, if the composition contains anionic surfactants, the level of such anionic surfactants is very low. In the present invention, a total level of such anionic surfactants, if included, alternatively 1% or less, alternatively 0.5% or less, alternatively 0.1% or less by weight of the composition. Most alternatively, the total level of such anionic surfactants is 0% by weight of the composition.

### ADDITIONAL COMPONENTS

The composition of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from about 0.001% to about 10%, alternatively up to about 5% by weight of the composition.

A wide variety of other additional components can be formulated into the present compositions. These include: other conditioning agents such as aloe vera gel; aloe barbadensis leaf juice; ecklonia radiata extract; natural oils and waxes with shea butter, safflower oil, cocoa butter, orange peel wax, olive oil, macadamia seed oil, oenothera biennis oil, crambe abyssinica see oil, argon oil, camelina oil, sunflower oil, almond oil, argania spinosa kernel oil, grape see oil, jojoba oil, coconut oil, meadowfoam seed oil, neem oil, linseed oil, castor oil, soybean oil, sesame oil, beeswax, sunflower wax, candelilla wax, rice bran wax, carnauba wax, bayberry wax and soy wax; essential oils such as lime peel oil, lavender oil, peppermint oil, cedarwood oil, tea tree oil, ylang-ylang oil and coensage oil which can be used in fragrance; hydrolyzed collagen with tradename Peptein 2000 available from Hormel, vitamin E with tradename Emix-d available from Eisai, panthenol available from Roche, panthenyl ethyl ether available from Roche, hydrolyzed keratin, proteins, plant extracts, and nutrients; pH adjusting agents, such as citric acid, sodium citrate, succinic acid, phosphoric acid, sodium hydroxide, sodium carbonate; salts, in general, such as potassium acetate and sodium chloride; coloring agents, such as any of the FD&C or D&C dyes; perfumes; and sequestering agents, such as disodium ethylenediamine tetra-acetate; and ultraviolet and infrared screening and absorbing agents such as octyl salicylate; antioxidants include: rosemary, tocopherol, vitamin E, vitamin A,tea extracts, and hydroxyacetophenone (available as SymSave^{®} H from Symrise^{®}); amino acids include histidine, 1-arginine and others.

### PRODUCT FORMS

The compositions of the present invention can be in the form of rinse-off products or leave-on products and can be formulated in a wide variety of product forms, including but not limited to creams, gels, emulsions, mousses, and sprays.

The conditioning composition of the present invention is especially suitable for rinse-off hair conditioner. Such compositions are alternatively used by following steps:
(i) after shampooing hair, applying to the hair an effective amount of the conditioning compositions for conditioning the hair; and
(ii) then rinsing the hair.

### TEST METHODS

### Bacterial Microbial Susceptibility Testing Method

Bacterial microbial susceptibility testing is used to assess the anti-bacterial effectiveness of the preservation system in cosmetic rinse-off conditioner.

A bacterial pool (mixture in equal volumes) of challenge organisms used in the test is comprised of standardized solutions of the bacterial strains *Escherichia coli* (ATCC# 8739), *Staphylococcus aureus* (ATCC# 6538), *Pseudomonas aeruginosa* (ATCC# 9027), *Burkholderia cepacia* (ATCC#25416), as well as *Klebsiella pneumoniae, Enterobacter gergoviae* and *Serratia marcescens strains* isolated from cosmetic products. The bacterial pool is prepared to have a concentration of approximately 6-8 log cfu/ml. To start the test, 0.1 ml of the bacterial pool is added into 10.0 g of a test conditioner. The test conditioner is then incubated for 2 days at 20-25°C. After incubation, a 1.0g aliquot of product is neutralized using Modified Letheen Broth containing 1.5% polysorbate 80 (commercially available as Tween^{®} 80 from Croda^{™}) and 1% Lecithin to aid in microbial recovery/enumeration. Then, multiple diluted concentrations of this sample are transferred into petri dishes containing Modified Letheen Agar with 1.5% Tween^{®} 80, and the agar plates are incubated at least 2 days at 30-35°C. Bacterial colony forming units (cfus) are then enumerated, and a bacterial log reduction from the starting log cfu/g challenge level is reported.

A 1 log cfu/g reduction equates to ~ a 90% bacterial reduction. A 2 log cfu/g reduction equates to ~ a 99% bacterial reduction. A 3 log cfu/g reduction equates to ~ a 99.9% bacterial reduction. A 4 log cfu/g reduction equates to ~ a 99.99% bacterial reduction. Greater log cfu/g reduction values indicate greater antimicrobial robustness from the preservation system.

### Fungal Microbial Susceptibility Testing Method:

Fungal microbial susceptibility testing is used to assess the anti-fungal effectiveness of the preservation system in cosmetic rinse-off conditioner.

Standardized ATCC strains of the yeast *Candida albicans* (ATCC# 10231) and mold *Aspergillus brasiliensis* (frm. *niger)* (ATCC# 16404) are mixed in 1:1 (v:v) ratio, and this fungal pool is used as inoculum in the test. The concentration of the fungal pool is approximately 6-8 log cfu/ml. To start the test, 0.1 ml of the fungal pool is added into 10.0 g of a testing conditioner. After the inoculated sample is incubated for 2 days at 20-25°C, a 1.0 g aliquot of product is neutralized using Modified Letheen Broth containing 1.5% Tween^{®} 80 and 1% Lecithin to aid in microbial recovery/enumeration. Then, multiple diluted concentrations of this sample are transferred into petri dishes containing Modified Letheen Agar with 1.5% Tween 80, and the agar plates are incubated for at least 5 days at 20-25°C, at which time fungal colony forming units (cfus) are then enumerated, and a fungal log reduction from the starting log cfu/g challenge level is calculated.
A 1 log cfu/g reduction equates to ~ a 90% fungal reduction. A 2 log cfu/g reduction equates to ~ a 99% fungal reduction. A 3 log cfu/g reduction equates to ~ a 99.9% fungal reduction. A 4 log cfu/g reduction equates to ~ a 99.99% fungal reduction. Greater log cfu/g reduction values indicate greater anti-fungal robustness from the preservation system.

### Differential Scanning Calorimetry

The melt transition behavior and temperature for the gel network may be obtained using differential scanning calorimetry (DSC) according to the following method. Utilizing a TA Instruments Q2000 DSC, approximately 15 mg of the gel network pre-mix or the final conditioner composition containing the gel network is placed into a Tzero aluminum hermetic DSC pan. The sample, along with an empty reference pan is placed into the instrument. The samples are analyzed using the following conditions/temperature program: Nitrogen Purge at a rate of 50.0 mL/min; Equilibrate @ 20.00 °C; Sampling interval 0.10 sec/pt; Equilibrate at 5.00°C; Isothermal for 1.00 min; Ramp 5.00°C/min to 80.00°C. The resulting DSC data is analyzed using TA Instruments Universal Analysis Software.

The use of DSC to measure the melt transition behavior and temperature for gel networks is further described by T. de Vringer et al., Colloid and Polymer Science, vol. 265, 448-457 (1987); and H. M. Ribeiro et al., Intl. J. of Cosmetic Science, vol. 26, 47-59 (2004).

### pH Method

First, calibrate the Mettler Toledo Seven Compact pH meter. Do this by turning on the pH meter and waiting for 30 seconds. Then take the electrode out of the storage solution, rinse the electrode with distilled water, and carefully wipe the electrode with a scientific cleaning wipe, such as a Kimwipe^{®}. Submerse the electrode in the pH 4 buffer and press the calibrate button. Wait until the pH icon stops flashing and press the calibrate button a second time. Rinse the electrode with distilled water and carefully wipe the electrode with a scientific cleaning wipe. Then submerse the electrode into the pH 7 buffer and press the calibrate button a second time. Wait until the pH icon stops flashing and press the calibrate button a third time. Rinse the electrode with distilled water and carefully wipe the electrode with a scientific cleaning wipe. Then submerse the electrode into the pH 10 buffer and press the calibrate button a third time. Wait until the pH icon stops flashing and press the measure button. Rinse the electrode with distilled water and carefully wipe with a scientific cleaning wipe.

Submerse the electrode into the testing sample and press the read button. Wait until the pH icon stops flashing and record the value.

### Shear Stress

Shear stress is measured by shear rate sweep condition with a rheometer available from TA Instruments with a mode name of ARG2. Geometry has 40 mm diameter, 2° C. cone angle, and gap of 49 µm. Shear rate is logarithmically increased from 0 to 1200/s for 1 min, and temperature is kept at 26.7° C. Share stress at a high shear rate of 950/s is measured and defined above.

### X-ray Diffraction Method

*SAXS (Small Angle X-ray Scattering)* is used to confirm the presence of a multi-lamellar phase, and *WAXS (Wide Angle X-ray Scattering)* is used to differentiate between Lα (liquid) and Lβ (solid) crystalline structures were employed to verify the presence of the characteristic dispersed gel network phase of the personal conditioning compositions

### d-spacing (Lβ-basal spacing) of lamella gel network:

Small-angle x-ray scattering ("SAXS") as used to resolve periodic structures in mesophases is essentially an x-ray diffraction technique. It is used in conjunction with conventional wide-angle x-ray scattering ("WAXS") to characterize aggregate structures such as micelles, gel networks, lamella, hexagonal and cubic liquid crystals. The different mesophases that show periodic structures can be characterized by the relative positions (d-spacing) of their reflections as derived from the Bragg equation (d=λ/2 Sin θ) where d represents the interplanar spacing, λ the radiation wavelength and θ the scattering (diffraction) angle.

The one dimensional lamella gel network phase is characterized by the ratio of the interplanar spacings d₁/d₁, d₁/d₂, d₁/d₃, d₁/d₄, d₁/d₅ having the values 1:2:3:4:5 etc. in the SAXS region (long-range order) and one or two invariant reflection(s) in the WAXS region (short-range) centered around 3.5 and 4.5 Å over a broad halo background. Other mesophases (e.g. hexagonal or cubic) will have characteristically different d-spacing ratios.

The SAXS data was collected with a Bruker NanoSTAR small-angle x-ray scattering instrument. The micro-focus Cu x-ray tube was operated at 50kV, 0.60mA with 550um ScanTex Pinholes. The sample to detector distance was 107.39 cm and the detector a Vantec2K 2-dimensional area detector. Samples were sealed in capillaries and analyzed under vacuum with an analysis time of 600s.

The value of d-spacing ((Lβ-basal spacing) of lamella gel network reported here is obtained with the 1^{st} order of SAXS reflection which is the d₁ spacing.

WAXS confirmation (in combination with SAXS) of presence of Lβ Gel Network

Wide-angle data (WAXS) was collected on a Stoe STADI-MP diffractometer. The generator was operated at 40kV/40mA, powering a copper anode long-fine-focus Cu x-ray tube. The diffractometer incorporates an incident-beam curved germanium-crystal monochromator, standard incident-beam slit system, and Mythen PSD detector. Data were collected in transmission mode over a range of 0° to 50° 2θ with a step size of 3° 2θ and 15 seconds per step.

WAXS Pattern with reflection near 4.2Å which, in combination with the lamellar reflections seen in the SAXS, is indicative of the presence of Lβ gel network.

### EXAMPLES

The following are non-limiting examples of the conditioner compositions described herein.

All parts, percentages, and ratios herein are by weight unless otherwise specified. Some components may come from suppliers as dilute solutions. The amount stated reflects the weight percent of the added material, unless otherwise specified.

The examples in Table 2 to Table 11 were made as follows. Sodium benzoate and ℓ-glutamic were dissolved in the water. The mixture was heated to 80°C. Then, the cationic surfactant and fatty alcohols (FAOH) were added to the mixture. Next, the mixture was cooled while the cationic surfactant and fatty alcohols continue to dissolve. Then, the additional preservatives were added followed by oils and perfume when the temperature was below 45°C. The composition was cooled to room temperature to make the conditioner composition.

Table 2, below, shows Comparative Examples 1-4. Even though these compositions contain an effective surfactant system that that is EWG VERIFIED^{™}, does not contain any of the ingredients that Whole Foods^{®} Market lists as unacceptable, and is categorized as "risk-free" by the Yuka^{®} Application, they are not consumer preferred.

**Table 2: Comparative Examples 1-2**

| | Comp. Ex. 1 | Comp. Ex. 2 | Comp. Ex. 3 | Comp. Ex. 4 |
|---|---|---|---|---|
| Sheer stress (Pa) @950 1/s | 60 | 65 | 354 | 299 |
| DSC peak (melting temp) | 2 peaks | 2 peaks | 2 peaks | 2 peaks |
| Behenamidopropyl Dimethylamine (BAPDMA) wt% (active)¹ | 6.74 | 7.48 | 2.25 | 1.50 |
| ℓ-Glutamic Acid wt% (active)² | 2.78 | 3.09 | 0.93 | 0.62 |
| Cetyl Alcohol (C16 Fatty alcohol) wt% (active)³ | 2.81 | 2.55 | 4.34 | 4.59 |
| Stearyl Alcohol (C18 Fatty Alcohol) wt% (active)⁴ | 2.09 | 1.90 | 3.23 | 3.42 |
| Sodium Benzoate wt% (active)⁵ | 0.20 | 0.20 | 0.20 | 0.20 |
| Caprylyl Glycol wt% (active)6 | 0.40 | 0.40 | 0.40 | 0.40 |
| Distilled Water | Q.S. | Q.S. | Q.S. | Q.S. |
| Citric Acid | Adjust pH to 3.5-4.5 | | | |
| Total GN content (BAPDMA+FAOH molar) | 0.351 | 0.351 | 0.351 | 0.351 |
| C18 FAOH/total FAOH (molar) | 0.40 | 0.40 | 0.40 | 0.40 |
| ℓ-Glutamic acid to BAPDMA molar ratio | 1.24 | 1.25 | 1.25 | 1.25 |
| Molar ratio of BAPDMA to FAOH | 45:55 | 50:50 | 15:85 | 10:90 |

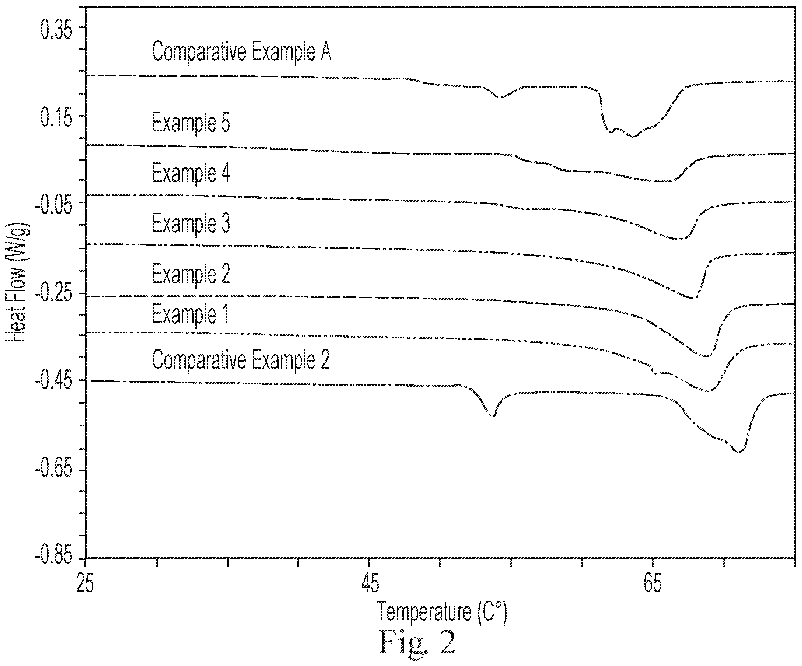

Comparative Examples 1-4 all have two peaks, according to the Differential Scanning Calorimetry Test Method, which indicates that the gel network is non-uniform and may provide poor conditioning, a poor user experience, and/or instability including a short shelf life. FIG. 2 shows the melt transition behavior of the gel network of select examples: Examples 1-5 (see Table 3, below) and Comparative Examples 1 and 2. The curves were made according to the Differential Scanning Calorimetry Test Method, described herein. The DSC curve for Comparative Examples 1 and 2 all show two peaks.

In Comparative Examples 1-2, the molar ratio of BAPDMA to fatty alcohol is too high and since there is an excess of BAPDMA, you do not form the correct gel network that provides good conditioning and shear stress to form a consumer acceptable product.

In Comparative Examples 3-4, the molar ratio BADPMA to fatty alcohol is too low and since there is too little BADPMA not enough fatty alcohol was incorporated into the gel network. These examples have a poor gel network that does not provide good conditioning. Also, the excess of fatty alcohol can leave a greasy or weighed down feeling to the hair because too much fatty alcohol is deposited on the hair.

Table 3 to Table 8, below, show Examples 1-35, which are consumer acceptable conditioner compositions. The uniform conditioner compositions in Table 3 to Table 8 contain an effective surfactant system that that is EWG VERIFIED^{™}, does not contain any of the ingredients that Whole Foods^{®} Market lists as unacceptable, and is categorized as "risk-free" by the Yuka^{®} Application.

**Table 3: Examples 1-6**

| | Ex. 1 | Ex. 2 | Ex. 3 | Ex. 4 | Ex. 5 | Ex. 6 |
|---|---|---|---|---|---|---|
| Sheer stress (Pa) @950 1/s | 278 | 334 | 345 | 160 | 107 | 270 |
| DSC peak (melting temp) | 1 peak | 1 peak | 1 peak | 1 peak | 1 peak | 1 peak |
| GN d-spacing (nm) | 24.8 | 20.8 | 23.9 | 22.8 | 21.2 | 26.8 |
| Behenamidopropyl Dimethylamine (BAPDMA) wt% (active)¹ | 2.99 | 3.74 | 4.50 | 5.24 | 5.99 | 2.99 |
| ℓ-Glutamic Acid wt% (active)² | 1.24 | 1.05 | 1.86 | 2.16 | 2.47 | 0.83 |
| Cetyl Alcohol (C16 Fatty alcohol) wt% (active)³ | 4.08 | 3.83 | 3.57 | 3.31 | 3.06 | - |
| Stearyl Alcohol (C18 Fatty Alcohol) wt% (active)⁴ | 3.03 | 2.84 | 2.66 | 2.46 | 2.28 | - |
| Brassical Alcohol (C21 Fatty Alcohol) wt%⁷ | - | - | - | - | - | 8.77 |
| Sodium Benzoate wt% (active)⁵ | 0.20 | 0.20 | 0.20 | 0.20 | 0.20 | 0.20 |
| Benzyl Alcohol wt% (active)⁸ | - | - | - | - | - | 0.40 |
| Shea Butter wt% (active)⁹ | 0.50 | 0.50 | 0.50 | 0.50 | 0.50 | 0.50 |
| Perfume | 0.65 | 0.65 | 0.65 | 0.65 | 0.65 | 0.65 |
| Distilled Water | Q.S. | Q.S. | Q.S. | Q.S. | Q.S. | Q.S. |
| Citric Acid | Adjust pH to 3.5-4.5 | | | | | |
| Total GN content (BAPDMA+FAOH molar) | 0.351 | 0.351 | 0.351 | 0.351 | 0.351 | 0.351 |
| C18OH/total FAOH (molar) | 0.40 | 0.40 | 0.40 | 0.40 | 0.40 | - |
| ℓ-Glutamic acid to BAPDMA molar ratio | 1.25 | 0.85 | 1.25 | 1.24 | 1.24 | 0.84 |
| Molar ratio of BAPDMA to FAOH | 20:80 | 25:75 | 30:70 | 35:65 | 40:60 | 20:80 |

**Table 4: Examples 7-12**

| | Ex. 7 | Ex. 8 | Ex. 9 | Ex. 10 | Ex. 11 | Ex. 12 |
|---|---|---|---|---|---|---|
| Sheer stress (Pa) @950 1/s | 249 | 233 | 141 | 516 | 562 | 338 |
| DSC peak (melting temp) | 1 peak | 1 peak | 1 peak | 1 peak | 1 peak | 1 peak |
| GN d-spacing (nm) | 25.8 | 24.2 | 24.4 | 21.6 | 21.5 | 22 |
| Behenamidopropyl Dimethylamine (BAPDMA) wt% (active)¹ | 3.12 | 3.74 | 4.37 | 4.68 | 4.68 | 5.61 |
| ℓ-Glutamic Acid wt% (active)² | 0.87 | 1.22 | 1.22 | 1.95 | 1.95 | 1.95 |
| Cetyl Alcohol (C16 Fatty alcohol) wt% (active)³ | 1.60 | 1.49 | 1.81 | 4.78 | 4.78 | 4.46 |
| Stearyl Alcohol (C18 Fatty Alcohol) wt% (active)⁴ | 4.15 | 3.87 | 3.12 | 3.56 | 3.56 | 3.32 |
| Sodium Benzoate wt% (active)⁵ | 0.20 | 0.20 | 0.20 | 0.20 | 0.20 | 0.20 |
| Benzyl Alcohol wt% (active)⁷ | 0.40 | 0.40 | 0.40 | - | - | - |
| Shea Butter wt% (active)⁹ | 0.50 | 0.50 | 0.50 | 2.00 | 0.50 | 0.50 |
| Safflower oil wt% (active)¹⁰ | - | - | - | - | 0.55 | 0.55 |
| Argon oil wt% (active)¹¹ | - | - | - | 1.50 | - | - |
| jojoba oil wt% (active)¹² | - | - | - | 1.50 | - | - |
| Perfume | 0.65 | 0.65 | 0.65 | 0.65 | 0.65 | 0.65 |
| Distilled Water | Q.S. | Q.S. | Q.S. | Q.S. | Q.S. | Q.S. |
| Citric Acid | Adjust pH to 3.5-4.5 | | | | | |
| Total GN content (BAPDMA+FAOH molar) | 0.293 | 0.293 | 0.293 | 0.439 | 0.439 | 0.439 |
| C18 FAOH/total FAOH (molar) | 0.70 | 0.70 | 0.70 | 0.40 | 0.40 | 0.40 |
| ℓ-Glutamic acid to BAPDMA molar ratio | 0.84 | 0.98 | 0.84 | 1.26 | 1.26 | 1.05 |
| Molar ratio of BAPDMA to FAOH | 25:75 | 30:70 | 35:65 | 25:75 | 25:75 | 30:70 |

**Table 5: Examples 13-18**

| | Ex. 13 | Ex. 14 | Ex. 15 | Ex. 16 | Ex. 17 | Ex. 18 |
|---|---|---|---|---|---|---|
| Sheer stress (Pa) @950 1/s | 456 | 290 | 158 | 174 | 276 | 275 |
| DSC peak (melting temp) | 1 peak | 1 peak | 1 peak | 1 peak | 1 peak | 1 peak |
| GN d-spacing (nm) | 21.5 | 25.4 | 24.2 | 26.2 | 24.9 | 23.5 |
| Behenamidopropyl Dimethylamine (BAPDMA) wt% (active)¹ | 4.21 | 3.74 | 3.74 | 3.74 | 3.74 | 3.74 |
| ℓ-Glutamic Acid wt% (active)² | 1.75 | 1.55 | 1.55 | 1.05 | 1.05 | 1.05 |
| Cetyl Alcohol (C16 Fatty alcohol) wt% (active)³ | 4.30 | 3.83 | 3.83 | 3.83 | 1.91 | 1.91 |
| Stearyl Alcohol (C18 Fatty Alcohol) wt% (active)⁴ | 3.20 | 2.84 | 2.84 | 2.84 | 4.99 | 4.99 |
| Sodium Benzoate wt% (active)⁵ | 0.20 | 0.20 | 0.20 | 0.20 | 0.20 | 0.20 |
| Caprylyl Glycol wt% (active)⁶ | - | - | 0.40 | 0.40 | - | - |
| Benzyl Alcohol wt% (active)⁷ | - | - | - | - | - | 0.40 |
| Shea Butter wt% (active)⁹ | 0.50 | 2.00 | 0.50 | 0.50 | 0.50 | 0.50 |
| Safflower oil wt% (active)¹⁰ | 0.55 | - | - | - | - | - |
| Argon oil wt% (active)¹¹ | - | 1.50 | - | - | - | - |
| jojoba oil wt% (active)¹² | - | 1.50 | - | - | - | - |
| Perfume | 0.65 | 0.65 | 0.65 | 0.65 | 0.65 | 0.65 |
| Distilled Water | Q.S. | Q.S. | Q.S. | Q.S. | Q.S. | Q.S. |
| Citric Acid | Adjust pH to 3.5-4.5 | | | | | |
| Total GN content (BAPDMA+FAOH molar) | 0.395 | 0.351 | 0.351 | 0.351 | 0.351 | 0.351 |
| C18 FAOH/total FAOH (molar) | 0.40 | 0.40 | 0.40 | 0.40 | 0.70 | 0.70 |
| ℓ-Glutamic acid to BAPDMA molar ratio | 1.25 | 1.25 | 1.25 | 0.85 | 0.85 | 0.85 |
| Molar ratio of BAPDMA to FAOH | 25:75 | 25:75 | 25:75 | 25:75 | 25:75 | 25:75 |

**Table 6: Examples 19-24**

| | Ex. 19 | Ex. 20 | Ex. 21 | Ex. 22 | Ex. 23 | Ex. 24 |
|---|---|---|---|---|---|---|
| Sheer stress (Pa) @950 1/s | 303 | 261 | 178 | 231 | 170 | 160 |
| DSC peak (melting temp) | 1 peak | 1 peak | 1 peak | 1 peak | 1 peak | 1 peak |
| GN d-spacing (nm) | 21.9 | 23.1 | 23.1 | 22.7 | 23.1 | 22.8 |
| Behenamidopropyl Dimethylamine (BAPDMA) wt% (active)¹ | 4.50 | 4.50 | 4.50 | 5.24 | 5.24 | 5.24 |
| ℓ-Glutamic Acid wt% (active)² | 1.86 | 1.25 | 1.25 | 1.46 | 1.46 | 1.46 |
| Cetyl Alcohol (C16 Fatty alcohol) wt% (active)³ | 1.79 | 1.70 | 1.79 | 3.32 | 1.66 | 1.66 |
| Stearyl Alcohol (C18 Fatty Alcohol) wt% (active)⁴ | 4.65 | 4.51 | 4.65 | 2.46 | 4.32 | 4.32 |
| Sodium Benzoate wt% (active)⁵ | 0.20 | 0.20 | 0.20 | 0.20 | 0.20 | 0.20 |
| Caprylyl Glycol wt% (active)⁶ | - | - | 0.40 | - | - | - |
| Benzyl Alcohol wt% (active)⁷ | - | 0.40 | - | 0.40 | - | 0.40 |
| Shea Butter wt% (active)⁹ | 0.50 | 0.50 | 0.50 | 0.50 | 0.50 | 0.50 |
| Perfume | 0.65 | 0.65 | 0.65 | 0.65 | 0.65 | 0.65 |
| Distilled Water | Q.S. | Q.S. | Q.S. | Q.S. | Q.S. | Q.S. |
| Citric Acid | Adjust pH to 3.5-4.5 | | | | | |
| Total GN content (BAPDMA+FAOH molar) | 0.351 | 0.351 | 0.351 | 0.351 | 0.351 | 0.351 |
| C18 FAOH/total FAOH (molar) | 0.70 | 0.70 | 0.70 | 0.40 | 0.70 | 0.70 |
| ℓ-Glutamic acid to BAPDMA molar ratio | 1.25 | 0.84 | 0.84 | 0.84 | 0.84 | 0.84 |
| Molar ratio of BAPDMA to FAOH | 30:70 | 30:70 | 30:70 | 20:101 | 20:102 | 20:103 |

**Table 7: Examples 25-28**

| | Ex. 25 | Ex. 26 | Ex. 27 | Ex. 28 |
|---|---|---|---|---|
| Sheer stress (Pa) @950 1/s | 258 | 283 | 287 | 300 |
| DSC peak (melting temp) | 1 peak | 1 peak | 1 peak | 1 peak |
| GN d-spacing (nm) | 23.2 | 21.2 | 23.1 | 22.3 |
| Behenamidopropyl Dimethylamine (BAPDMA) wt% (active)¹ | 3.74 | 3.74 | 3.74 | 3.74 |
| ℓ-Glutamic Acid wt% (active)² | 1.55 | 1.55 | 1.55 | 1.55 |
| Cetyl Alcohol (C16 Fatty alcohol) wt% (active)³ | 3.83 | 3.83 | 3.83 | 3.83 |
| Stearyl Alcohol (C18 Fatty Alcohol) wt% (active)⁴ | 2.84 | 2.84 | 2.84 | 2.84 |
| Sodium Benzoate wt% (active)⁵ | 0.20 | 0.20 | 0.20 | 0.20 |
| Shea Butter wt% (active)⁹ | 0.50 | 0.50 | - | - |
| Safflower oil wt% (active)¹⁰ | - | 0.55 | 2.00 | - |
| Histidine wt% (active)¹³ | 0.01 | 0.01 | 0.01 | 0.01 |
| Aloe Barbadensis Leaf Juice wt% (active)¹⁴ | 0.05 | 0.05 | 0.05 | 0.05 |
| Ecklonia Radiata Extract wt% (active)¹⁵ | 0.02 | 0.02 | 0.02 | 0.02 |
| Perfume | 0.65 | 0.65 | 0.65 | 0.40 |
| Distilled Water | Q.S. | Q.S. | Q.S. | Q.S. |
| Citric Acid | Adjust pH to 3.5-4.5 | | | |
| Total GN content (BAPDMA+FAOH molar) | 0.351 | 0.351 | 0.351 | 0.351 |
| C18 FAOH/total FAOH (molar) | 0.40 | 0.40 | 0.40 | 0.40 |
| ℓ-Glutamic acid to BAPDMA molar ratio | 1.25 | 1.25 | 1.25 | 1.25 |
| Molar ratio of BAPDMA to FAOH | 25:75 | 25:75 | 25:75 | 25:75 |

**Table 8: Examples 29-35**

| | Ex. 29 | Ex. 30 | Ex. 31 | Ex. 32 | Ex. 33 | Ex. 34 | Ex. 35 |
|---|---|---|---|---|---|---|---|
| Sheer stress (Pa) @950 1/s | 306 | 312 | 150 | 172 | 117 | 118 | 198 |
| Behenamidopropyl Dimethylamine (BAPDMA) wt% (active)¹ | 3.74 | 3.74 | 3.72 | 3.72 | 3.27 | 3.27 | 2.81 |
| ℓ-Glutamic Acid wt% (active)² | 1.55 | 1.55 | 1.50 | 1.55 | 0.91 | 0.91 | 0.78 |
| Cetyl Alcohol (C16 Fatty alcohol) wt% (active)³ | 3.83 | 3.83 | 1.50 | 2.47 | 1.26 | 2.53 | 1.36 |
| Stearyl Alcohol (C18 Fatty Alcohol) wt% (active)⁴ | 2.84 | 2.84 | 2.86 | 1.80 | 2.45 | 1.33 | 2.64 |
| Sodium Benzoate wt% (active)⁵ | 0.2 | 0.25 | 0.25 | 0.25 | 0.25 | 0.25 | 0.25 |
| Caprylyl Glycol wt% (active)⁶ | 0.40 | 0.40 | 0.40 | 0.40 | 0.40 | 0.40 | 0.40 |
| Histidine wt% (active)¹³ | 0.01 | 0.01 | 0.01 | 0.01 | 0.01 | 0.01 | 0.01 |
| Aloe Barbadensis Leaf Juice wt% (active)¹⁴ | 0.05 | 0.05 | 0.05 | 0.05 | 0.05 | 0.05 | 0.05 |
| Ecklonia Radiata Extract wt% (active)¹⁵ | 0.02 | 0.02 | 0.02 | 0.02 | 0.02 | 0.02 | 0.02 |
| Perfume | 0.40 | 0.40 | 0.40 | 0.40 | 0.40 | 0.40 | 0.40 |
| Distilled Water | Q.S. | Q.S. | Q.S. | Q.S. | Q.S. | Q.S. | Q.S. |
| Citric Acid | Adjust pH to 3.5-4.5 | | | | | | |
| Total molar content of BAPDMA+FAOH | 0.351 | 0.351 | 0.249 | 0.249 | 0.220 | 0.220 | 0.220 |
| C18 FAOH/total FAOH (molar) | 0.40 | 0.40 | 0.70 | 0.40 | 0.70 | 0.40 | 0.70 |
| ℓ-Glutamic acid to BAPDMA molar ratio | 1.25 | 1.25 | 1.22 | 1.26 | 0.84 | 0.84 | 0.84 |
| Molar ratio of BAPDMA to FAOH | 25:75 | 25:75 | 35:65 | 35:65 | 35:65 | 35:65 | 30:70 |

### Suppliers for Examples in Table 2 to Table 8

1. Behenamidopropyl Dimethylamine (BAPDMA) (Incromine^{™} BD), available from Croda^{®}
2. ℓ-Glutamic Acid, available from Ajinomoto^{®}
3. Cetyl alcohol, 95% active level available from Procter & Gamble^{®}
4. Stearyl alcohol, 97% active level, available from Procter & Gamble^{®}
5. Sodium Benzoate, available from Kalama^{®}
6. Caprylyl Glycol, available from Procter and Gamble^{®}
7. Brassical Alcohol (SustOleo^{™} BA), available from Inolex^{®}
8. Benzyl Alcohol, available form Charkit^{®}
9. Shea Butter, available form Procter and Gamble^{®}
10. Safflower oil (*Carthamus tinctorius* seed oil), available from Southern Cross Botanicals
11. Argon oil (*Argania spinosa* kernel oil), available from BASF^{®}
12. Jojoba oil (*Simmondsia chinensis* seed oil), available from Southern Cross Botanicals
13. Histidine (L-Histidine), available from Ajinomoto^{®}
14. Aloe Barbadensis Leaf Juice, available form Procter and Gamble^{®}
15. Ecklonia Radiata Extract (Australian See Kelp Extract), available from Southern Cross Botanicals

Examples 1-35 all have one melting transition peak, according to the Differential Scanning Calorimetry Test Method, described herein, which indicates that the gel network is uniform and the may provide good conditioning performance, a good user experience, and may be stable. FIG. 2 shows the melt transition behavior of the gel network of Examples 1-5 (Table 3) and each curve for Examples 1-5 has only one peak. As shown in FIG. 2, Examples 1-5, one peak can be a single distinct peak, or it can be merged set of peaks. However, Comparative Example 2 (Table 2) has more than one peak, which can indicate that the gel network is not uniform.

The shear stress is consumer acceptable and ranges from 107 Pa to 562 Pa, if the shear stress is too low or too high it can be difficult for a consumer to apply the conditioner composition throughout their hair with their hands. If the shear stress is too low, the conditioner composition can drip from the hand and hair and if the shear stress is too high, it can be difficult to spread.

The gel network d-spacing is from 20.8 to 26.9 for Examples 1-28. This level of d-spacing indicates that the conditioner composition can provide good conditioning with good wet feel and good wet detangling.

Examples 1-35 have a molar ratio of BAPDMA to FAOH of greater than or equal to 20:80 and less than or equal to 40:60. It was surprisingly found that if the molar ratio of BAPDMA to FAOH was in this range and the conditioner contains sodium benzoate then the conditioner composition had a gel network with d-spacing that indicates good wet conditioning performance was formed. It is believed that other salts, including sodium salicylate, sodium chloride, sodium carbonate, sodium borate, sodium acetate, sodium citrate, potassium benzoate, potassium acetate, calcium gluconate, calcium chloride, and potassium sorbate, would form a gel network with the proper d-spacing was formed.

In Table 9 to Table 11, below, the Micro - Bacteria @ 2 days and the Micro - Fungi @ 2 days is determined by the Bacterial and Fungal Microbial Susceptibility Test Methods, described herein. For the preservation system to be effective, the level of microbes (bacteria and fungi) needs to be undetectable, which means that there is a greater than 99.99% reduction in microbes at two days as determined by the Bacterial and Fungal Microbial Susceptibility Test Methods. In Table 10, below, N/A indicates that the test was not performed.

**Table 9: Comp. Examples A-F**

| | Comp. Ex. A | Comp. Ex. B | Comp. Ex. C | Comp. Ex. D | Comp. Ex. E | Comp. Ex. F |
|---|---|---|---|---|---|---|
| Appearance | Creamy and smooth | | | | | |
| Sheer stress (Pa) @950 1/s | 350 | 419 | 448 | 450 | 354 | 343 |
| Micro - Bacteria @ 2 days | ~90% reduction | Not Detectable (>99.99% reduction) | | | | |
| Micro - Fungi @ 2 days | ~90% reduction | | | >99% reduction | | |
| Sodium Benzoate wt% (active) ¹ | - | 0.20 | 0.25 | 0.40 | 0.20 | 0.25 |
| Caprylyl Glycol wt% (active) ² | - | - | - | - | 0.20 | 0.20 |
| Behenamidopropyl Dimethylamine (Cationic Surfactant) wt% (active)⁶ | 3.74 | 3.74 | 3.74 | 3.74 | 3.74 | 3.74 |
| ℓ-Glutamic Acid wt% (active)⁷ | 1.55 | 1.55 | 1.55 | 1.55 | 1.55 | 1.55 |
| Cetyl Alcohol (C16 Fatty alcohol) wt% (active)⁸ | 3.83 | 3.83 | 3.83 | 3.83 | 3.83 | 3.83 |
| Stearyl Alcohol (C18 Fatty Alcohol) wt% (active)⁹ | 2.84 | 2.84 | 2.84 | 2.84 | 2.84 | 2.84 |
| Citric Acid wt% | 0.25 | 0.25 | 0.35 | 0.25 | 0.25 | 0.35 |
| Distilled Water | Q.S. | Q.S. | Q.S. | Q.S. | Q.S. | Q.S. |
| pH | 3.9 | 4.2 | 4.2 | 4.3 | 4.2 | 4.2 |

**Table 10: Comparative Examples G-K**

| | Comp. Ex. G | Comp. Ex. H | Comp. Ex. I | Comp. Ex. J | Comp. Ex. K |
|---|---|---|---|---|---|
| Appearance | Creamy and smooth | | | Clumpy and grainy | Thin and grainy |
| Sheer stress (Pa) @950 1/s | 270 | 275 | 271 | N/A | |
| Micro - Bacteria @ 2 day | Not Detectable (>99.99% reduction) | | | N/A | |
| Micro - Fungi @ 2 days | >90% reduction | | >99% reduction | N/A | |
| Sodium Benzoate wt% (active)¹ | 0.20 | 0.20 | 0.20 | 1.00 | - |
| Caprylyl Glycol wt% (active)² | - | - | - | - | 2.00 |
| Glyceryl Caprylate/Caprate wt% (active)³ | 0.40 | - | - | - | - |
| Glyceryl Caprylate (and) Glyceryl Undecylenate wt% (active)⁴ | - | 0.40 | - | - | - |
| Behentrimonium Chloride (Cationic Surfactant) wt% (active)⁵ | - | - | 2.28 | - | - |
| Behenamidopropyl Dimethylamine | 3.74 | 3.74 | - | 3.74 | 3.74 |
| (cationic surfactant) wt% (active)⁶ | | | | | |
| ℓ-Glutamic Acid wt% (active)⁷ | 1.55 | 1.55 | - | 1.55 | 1.55 |
| Cetyl Alcohol (C16 Fatty alcohol) wt% (active)⁸ | 3.83 | 3.83 | 1.67 | 3.83 | 3.83 |
| Stearyl Alcohol (C18 Fatty Alcohol) wt% (active) ⁹ | 2.84 | 2.84 | 4.50 | 2.84 | 2.84 |
| Citric Acid | 0.25 | 0.25 | 0.035 | 1.25 | 0.25 |
| Distilled Water | Q.S. | Q.S. | Q.S. | Q.S. | Q.S. |
| pH | 4 | 4.1 | 4.4 | 4 | 4.1 |

Comparative Example A is the control and does not contain a preservation system. Comparative Example A does not provide enough microbe reduction at 2 days for bacteria and fungi.

Comparative Examples B, C, and D contain 0.20 wt%, 0.25 wt%, and 0.40 wt% sodium benzoate, respectively, and these examples have an undetectable level (>99.99% reduction) of bacteria at two days. However, a preservation system that contains only sodium benzoate at these levels does not provide enough fungi reduction at two days, as these examples only have a ~90% reduction.

Comparative Examples E and F include a preservation system that has both sodium benzoate and caprylyl glycol. These examples have an undetectable level (>99.99% reduction) of bacteria at two days. The combination of sodium benzoate and caprylyl glycol improves the reduction of fungi at 2 days, as compared to Comparative Examples B, C, and D. However, there is still a detectable level of fungi and therefore the preservation system in these examples is not considered effective.

Comparative Example G has a preservation system with 0.20 wt% sodium benzoate and 0.40 wt% glyceryl caprylate/caprate (a glyceryl ester) and this example has an undetectable level (>99.99% reduction) of bacteria at two days. However, it has a >90% reduction in fungi at two days and is ineffective.

Similar to Comparative Example GG, Comparative Example H has a preservation system with 0.20 wt% sodium benzoate and 0.40 wt% glyceryl caprylate (and) glyceryl undeylenate and this example has an undetectable level (>99.99% reduction) of bacteria at two days. However, it has a >90% reduction in fungi at two days and is ineffective.

Comparative Example I contains 0.20% sodium benzoate and behentrimonium chloride (cationic surfactant) and this example has an undetectable level (>99.99% reduction) of bacteria at two days. However, it has a detectable level of fungi (>99% reduction) at two days and is therefore ineffective.

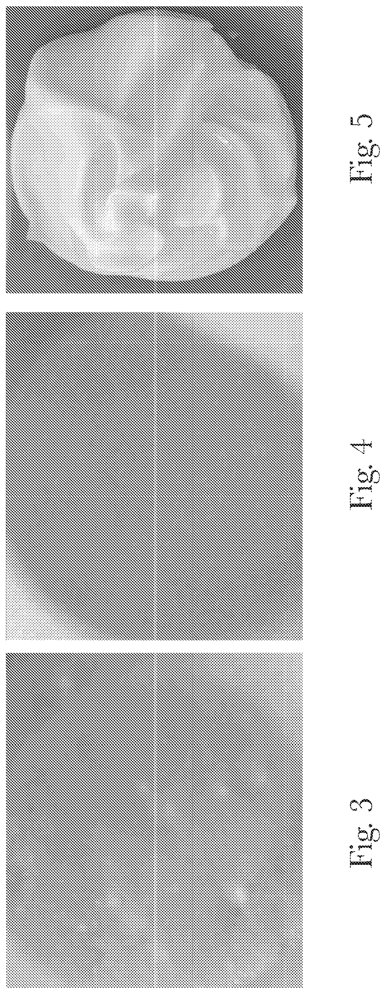

Comparative Example J contains 1 wt% sodium benzoate. As shown in FIG. 3, instead of being a smooth, creamy conditioner, it was clumpy and grainy, which is not consumer preferred. While not willing to be bound by theory, it is believed that the sodium benzoate level in this example is too high and since sodium benzoate is a salt, it effects the packing of the gel network. For example, in this example it is believed that some regions the gel network may be packed too tight and this can lead to the clumpy texture.

Comparative Example K contains 2 wt% caprylyl glycol. As shown in FIG. 4, instead of being a smooth, creamy conditioner, it was thin and crystalline appearance. A conditioner with this texture could be difficult for a consumer to apply with their hands and may not provide good conditioning. While not willing to be bound by theory, it is believed that if the level of glycol is too high, it acts like a solvent and breaks down the gel network.

**Table 11: Examples A-E**

| | Ex. A | Ex. B | Ex. C | Ex. D | Ex. E |
|---|---|---|---|---|---|
| Appearance | Creamy and smooth | | | | |
| Sheer stress (Pa) @950 1/s | 304 | 302 | 289 | 290 | 141 |
| Micro - Bacteria @ 2 days | Not Detectable (>99.99% reduction) | | | | |
| Micro - Fungi @ 2 days | Not Detectable (>99.99% reduction) | | | | |
| Sodium Benzoate wt% (active)¹ | 0.20 | 0.25 | 0.40 | 0.40 | 0.20 |
| Caprylyl Glycol wt% (active)² | 0.40 | 0.40 | - | - | 0.40 |
| Glyceryl Caprylate/Caprate wt% (active)³ | - | - | 0.40 | - | - |
| Glyceryl Caprylate (and) Glyceryl Undecylenate wt% (active)⁴ | - | - | - | 0.40 | - |
| Behentrimonium Chloride (Cationic Surfactant) wt% (active)⁵ | - | - | - | - | 2.28 |
| Behenamidopropyl Dimethylamine (Cationic Surfactant) wt% (active)⁶ | 3.74 | 3.74 | 3.74 | 3.74 | - |
| ℓ-Glutamic Acid wt% (active)⁷ | 1.55 | 1.55 | 1.55 | 1.55 | - |
| Cetyl Alcohol (C16 Fatty alcohol) wt% (active)⁸ | 3.83 | 3.83 | 3.83 | 3.83 | 1.67 |
| Stearyl Alcohol (C18 Fatty Alcohol) wt% (active)⁹ | 2.84 | 2.84 | 2.84 | 2.84 | 4.50 |
| Citric Acid | 0.25 | 0.35 | 0.25 | 0.25 | 0.035 |
| Distilled Water | Q.S. | Q.S. | Q.S. | Q.S. | Q.S. |
| pH | 4.2 | 4.2 | 4.1 | 4.1 | 4.4 |

### Suppliers for Examples in Table 9 to Table 11

1. Sodium Benzoate, available from Kalama^{®}
2. Caprylyl Glycol, available from Procter and Gamble^{®}
3. Glyceryl Caprylate/Caprate (STEPAN-MILD^{®} GCC), available from Stepan^{®}
4. Glyceryl Caprylate (and) Glyceryl Undecylenate (*Lexgard*^{®} *Natural*), available from Inolex^{®}
5. Behentrimonium Chloride/IPA (Genamin KDMP), available from Clariant^{™} at 80% active level
6. Behenamidopropyl Dimethylamine (BAPDMA) (Incromine^{™} BD), available from Croda^{®}
7. ℓ-Glutamic Acid, available from Ajinomoto^{®}
8. Cetyl alcohol, 95% active level available from Procter & Gamble^{®}
9. Stearyl alcohol, 97% active level, available from Procter & Gamble^{®}

All of the Examples in Table 11 have preservation systems that are effective (i.e. bacteria and fungi are not detectable (>99.99% reduction) at 2 days) and a creamy and smooth appearance that is consumer preferred. FIG. 5 is a photograph of Example A that shows the smooth and creamy appearance of the conditioner composition. These examples have between 0.20-0.40 wt% sodium benzoate and 0.40 wt% caprylyl glycol, glyceryl caprylate/caprate, or glyceryl caprylate (and) glyceryl undecylenate. The weight ratio of sodium benzoate to caprylyl glycol, glyceryl caprylate/caprate, or glyceryl caprylate (and) glyceryl undecylenate is from about 1:2 to about 1:1.

All the Examples in Table 11 have a creamy and smooth appearance that can be consumer preferred.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A hair conditioner composition comprising:
a. an aqueous carrier;
b. from 2.75 wt% to 6 wt% of behenamidopropyl dimethylamine;
c. from 3 wt% to 8 wt% of one or more fatty alcohols, preferably from 3.25 wt% to 7.5 wt% of one or more fatty alcohol, more preferably from 3.5 wt% to 7 wt% of one or more fatty alcohol, and most preferably from 4 wt% to 6.7 wt% of one or more fatty alcohol, wherein the one or more fatty alcohols have from 14 to 30 carbon atoms;
d. less than 1.5 wt% of a salt, preferably less than 1 wt% of the salt, more preferably less than 0.8 wt% of the salt, even more preferably less than 0.6 wt% of the salt, and most preferably less than 0.5 wt% of the salt; wherein the salt is selected from the group consisting of sodium benzoate, sodium salicylate, sodium chloride, sodium carbonate, sodium borate, sodium acetate, sodium citrate, potassium benzoate, potassium acetate, calcium gluconate, calcium chloride, and combinations thereof;
wherein the molar ratio of behenamidopropyl dimethylamine to fatty alcohol is from 9:50 to 2:3;
wherein the composition comprises a uniform gel network, wherein the conditioner composition comprises one peak, as measured according to the Differential Scanning Calorimetry Test Method;
wherein the composition comprises d-spacing from 10 nm to 32 nm, preferably from 15 nm to 30 nm, more preferably from 17 nm to 29 nm, and most preferably from 20 nm to 27 nm, as measured according to the d-spacing (Lβ-basal spacing) of Lamella Gel Network Test Method described herein.

2. The composition according to claim 1, wherein the one or more fatty alcohols is selected from the group consisting of stearyl alcohol, brassical alcohol, cetyl alcohol, and combinations thereof.

3. The composition according to according to any of the preceding claims, further comprising from 0.25 wt% to 6 wt% of an acid, preferably from 0.4 wt% to 5 wt% of an acid, more preferably from 0.5 wt% to 4 wt% of an acid, and most preferably from 0.6 wt% to 3 wt% of an acid; wherein the acid is selected from the group consisting l-glutamic acid, lactic acid, hydrochloric acid, malic acid, succinic acid, acetic acid, fumaric acid, tartaric acid, citric acid, l-glutamic hydrochloride, maleic acid, and mixtures thereof.

4. The composition according to any of the preceding claims, further comprising a gel network content of from 0.1 to 0.6 molar, preferably from 0.2 to 0.5 molar, and more preferably from 0.3 to 0.4 molar.

5. The composition according to any of the preceding claims, comprising a shear stress of from 75 Pa to 575 Pa, preferably 100 Pa to 565 Pa, more preferably from 105 Pa to 550 Pa, and most preferably from 125 Pa to 450 Pa, according to the Shear Stress Test Method described herein.

6. The hair conditioner composition according to any of the preceding claims, wherein the composition comprises less than 0.5% of behentrimonium chloride, cetrimonium chloride, and/or stearamidopropyl dimethylamine by weight of the composition.

7. The hair conditioner composition according to according to any of the preceding claims, wherein the composition comprises less than 0.5% by weight of the composition or is free of mono long alkyl quaternized ammonium salts.

8. The hair conditioner composition according to according to any of the preceding claims, wherein the composition comprises less than 0.5% of an ingredient by weight of the composition, wherein the ingredient is selected from the group consisting of silicone, propellants, phthalates, dyes, sulfates, formaldehyde donors, and combinations thereof.

9. The conditioner composition according to according to any of the preceding claims, wherein the composition comprises a pH from 2.5 to 5, and preferably from 3.5 to 4.5, as measured according to the pH Test Method, described herein.

10. The conditioner composition according to according to any of the preceding claims, further comprising a preservation system comprising:
a. from 0.1% to 0.5% of sodium benzoate, preferably from 0.2% to 0.4% of sodium benzoate, by weight of the composition;
b. from 0.3 wt% to 1.5 wt% of a second composition, preferably from 0.32 wt% to 1 wt% of a second composition, more preferably from 0.33 wt% to 0.8 wt% of a second composition, and even more preferably from 0.37 wt% to 0.45 wt% of a second composition; wherein the second composition is selected from the group consisting of a glycol, a glyceryl ester, and combinations thereof; wherein the ratio of sodium benzoate to the second composition is 1:4 to 1:1, preferably from 1:3 to 1:1, more preferably from 1:2 to 1:1, and even more preferably from 1:1.7 to 1:1.

11. The hair conditioner composition according to claim 10, wherein the second composition comprises a glycol, wherein the glycol is selected from the group consisting of butylene glycol, pentylene glycol, hexylene glycol, 1,2-hexanediol, caprylyl glycol, decylene glycol, and mixtures thereof.

12. The hair conditioner composition according to claim 10 or 11, wherein the second composition comprises a glyceryl ester, wherein the glyceryl ester is selected from the group consisting of glyceryl caprylate, glyceryl caprate, glyceryl undecylenate and mixtures thereof.

13. The hair conditioner composition according to claim 10, wherein the level of microbes is undetectable at two days, as measured according to the Bacterial and Fungal Microbial Susceptibility Test Methods.

14. Use of a hair conditioner composition according to any one of the preceding claims for providing satisfactory conditioning benefits in terms of wet feel and wet detangling.

## Patentansprüche

1. Haarspülungszusammensetzung, umfassend:
a. einen wässrigen Träger;
b. von zu 2,75 Gew.-% bis 6 Gew.-% Behenamidopropyldimethylamin;
c. von zu 3 Gew.-% bis 8 Gew.-% einen oder mehrere Fettalkohole, vorzugsweise von zu 3,25 Gew.-% bis 7,5 Gew.-% einen oder mehrere Fettalkohole, mehr bevorzugt von zu 3,5 Gew.-% bis 7 Gew.-% einen oder mehrere Fettalkohole und am meisten bevorzugt von zu 4 Gew.-% bis 6,7 Gew.-% einen oder mehrere Fettalkohole, wobei der eine oder die mehreren Fettalkohole von 14 bis 30 Kohlenstoffatome aufweisen;
d. zu weniger als 1,5 Gew.-% ein Salz, vorzugsweise zu weniger als 1 Gew.-% das Salz, mehr bevorzugt zu weniger als 0,8 Gew.-% das Salz, noch mehr bevorzugt zu weniger als 0,6 Gew.-% das Salz und am meisten bevorzugt zu weniger als 0,5 Gew.-% das Salz; wobei das Salz ausgewählt ist aus der Gruppe bestehend aus Natriumbenzoat, Natriumsalicylat, Natriumchlorid, Natriumcarbonat, Natriumborat, Natriumacetat, Natriumcitrat, Kaliumbenzoat, Kaliumacetat, Calciumgluconat, Calciumchlorid und Kombinationen davon;
wobei das Molverhältnis von Behenamidopropyldimethylamin zu Fettalkohol von 9 : 50 bis 2 : 3 beträgt;
wobei die Zusammensetzung ein gleichmäßiges Gelnetzwerk umfasst, wobei die Spülungszusammensetzung einen Peak umfasst, wie nach dem Prüfverfahren einer differentialen Scanningkalorimetrie gemessen;
wobei die Zusammensetzung einen d-Abstand von 10 nm bis 32 nm, vorzugsweise von 15 nm bis 30 nm, mehr bevorzugt von 17 nm bis 29 nm und am meisten bevorzugt von 20 nm bis 27 nm umfasst, wie nach dem d-Abstand (Lβ-Basalabstand) eines hierin beschriebenen Lamellengelnetzwerkprüfverfahrens gemessen.

2. Zusammensetzung nach Anspruch 1, wobei der eine oder die mehreren Fettalkohole ausgewählt sind aus der Gruppe bestehend aus Stearylalkohol, Brassica-Alkohol, Cetylalkohol und Mischungen davon.

3. Zusammensetzung nach einem der vorstehenden Ansprüche, ferner umfassend von zu 0,25 Gew.-% bis 6 Gew.-% eine Säure, vorzugsweise von zu 0,4 Gew.-% bis 5 Gew.-% eine Säure, mehr bevorzugt von zu 0,5 Gew.-% bis 4 Gew.-% eine Säure und am meisten bevorzugt von zu 0,6 Gew.-% bis 3 Gew.-% eine Säure; wobei die Säure ausgewählt ist aus der Gruppe bestehend aus I-Glutaminsäure, Milchsäure, Salzsäure, Äpfelsäure, Bernsteinsäure, Essigsäure, Fumarsäure, Weinsäure, Citronensäure, I-Glutaminhydrochlorid, Maleinsäure und Mischungen davon.

4. Zusammensetzung nach einem der vorstehenden Ansprüche, ferner umfassend einen Gelnetzwerkgehalt von 0,1 bis 0,6 molar, vorzugsweise von 0,2 bis 0,5 molar und mehr bevorzugt von 0,3 bis 0,4 molar.

5. Zusammensetzung nach einem der vorstehenden Ansprüche, umfassend eine Scherkraft von 75 Pa bis 575 Pa, vorzugsweise 100 Pa bis 565 Pa, mehr bevorzugt von 105 Pa bis 550 Pa und am meisten bevorzugt von 125 Pa bis 450 Pa, nach dem hierin beschriebenen Scherkraftprüfverfahren.

6. Haarspülungszusammensetzung nach einem der vorstehenden Ansprüche, wobei die Zusammensetzung zu weniger als 0,5 % Behentrimoniumchlorid, Cetrimoniumchlorid und/oder Stearamidopropyldimethylamin, bezogen auf das Gewicht der Zusammensetzung, umfasst.

7. Haarspülungszusammensetzung nach einem der vorstehenden Ansprüche, wobei die Zusammensetzung zu weniger als 0,5 %, bezogen auf das Gewicht der Zusammensetzung, mit monolangem Alkyl quaternisierte Ammoniumsalze umfasst oder frei von diesen ist.

8. Haarspülungszusammensetzung nach einem der vorstehenden Ansprüche, wobei die Zusammensetzung zu weniger als 0,5 % einen Bestandteil, bezogen auf das Gewicht der Zusammensetzung, umfasst, wobei der Bestandteil ausgewählt ist aus der Gruppe bestehend aus Silikon, Treibstoffen, Phthalaten, Farbstoffen, Sulfaten, Formaldehyddonatoren und Kombinationen davon.

9. Spülungszusammensetzung nach einem der vorstehenden Ansprüche, wobei die Zusammensetzung einen pH-Wert von 2,5 bis 5 und vorzugsweise von 3,5 bis 4,5 umfasst, wie nach dem hierin beschriebenen pH-Wertprüfverfahren gemessen.

10. Spülungszusammensetzung nach einem der vorstehenden Ansprüche, ferner umfassend ein Konservierungssystem, umfassend:
a. von zu 0,1 % bis 0,5 % Natriumbenzoat, vorzugsweise von zu 0,2 % bis 0,4 % Natriumbenzoat, bezogen auf das Gewicht der Zusammensetzung;
b. von zu 0,3 Gew.-% bis 1,5 Gew.-% eine zweite Zusammensetzung, vorzugsweise von zu 0,32 Gew.-% bis 1 Gew.-% eine zweite Zusammensetzung, mehr bevorzugt von zu 0,33 Gew.-% bis 0,8 Gew.-% eine zweite Zusammensetzung und noch mehr bevorzugt von zu 0,37 Gew.-% bis 0,45 Gew.-% eine zweite Zusammensetzung; wobei die zweite Zusammensetzung ausgewählt ist aus der Gruppe bestehend aus einem Glycol, einem Glycerylester und Kombinationen davon; wobei das Verhältnis von Natriumbenzoat zu der zweiten Zusammensetzung 1 : 4 bis 1 : 1, vorzugsweise von 1 : 3 bis 1 : 1, mehr bevorzugt von 1 : 2 bis 1 : 1 und noch mehr bevorzugt von 1 : 1,7 bis 1 : 1 beträgt.

11. Haarspülungszusammensetzung nach Anspruch 10, wobei die zweite Zusammensetzung ein Glycol umfasst, wobei das Glycol ausgewählt ist aus der Gruppe bestehend aus Butylenglycol, Pentylenglycol, Hexylenglycol, 1,2-Hexandiol, Caprylylglycol, Decylenglycol und Mischungen davon.

12. Haarspülungszusammensetzung nach Anspruch 10 oder 11, wobei die zweite Zusammensetzung einen Glycerylester umfasst, wobei der Glycerylester ausgewählt ist aus der Gruppe bestehend aus Glycerylcaprylat, Glycerylcaprat, Glycerylundecylenat und Mischungen davon.

13. Haarspülungszusammensetzung nach Anspruch 10, wobei der Mikrobenspiegel nach zwei Tagen nicht nachweisbar ist, wie nach den Prüfverfahren einer mikrobiellen Empfindlichkeit gegenüber Bakterien und Pilzen gemessen.

14. Verwendung einer Haarspülungszusammensetzung nach einem der vorstehenden Ansprüche zum Bereitstellen zufriedenstellender Konditionierungsnutzwirkungen hinsichtlich eines Nassgefühls und einer Nassentwirrung.

## Revendications

1. Composition d'après-shampoing comprenant :
a. un véhicule aqueux ;
b. de 2,75 % en poids à 6 % en poids de béhénamidopropyl diméthylamine ;
c. de 3 % en poids à 8 % en poids d'un ou de plusieurs alcools gras, de préférence de 3,25 % en poids à 7,5 % en poids d'un ou de plusieurs alcools gras, plus préférablement de 3,5 % en poids à 7 % en poids d'un ou de plusieurs alcools gras, et le plus préférablement encore de 4 % en poids à 6,7 % en poids d'un ou de plusieurs alcools gras, dans laquelle le ou les alcools gras ont de 14 à 30 atomes de carbone ;
d. moins de 1,5 % en poids d'un sel, de préférence moins de 1 % en poids du sel, de préférence moins de 0,8 % en poids du sel, encore plus préférablement encore moins de 0,6 % en poids du sel, et le plus préférablement moins de 0,5 % en poids du sel ; dans laquelle le sel est choisi dans le groupe constitué de benzoate de sodium, salicylate de sodium, chlorure de sodium, carbonate de sodium, borate de sodium, acétate de sodium, citrate de sodium, benzoate de potassium, acétate de potassium, gluconate de calcium, chlorure de calcium et combinaisons de ceux-ci ;
dans laquelle le rapport molaire entre la béhénamidopropyl diméthylamine et l'alcool gras est compris entre 9:50 et 2:3 ;
dans laquelle la composition comprend un réseau de gel uniforme, dans laquelle la composition de conditionnement comprend un pic, tel que mesuré selon le procédé d'essai de calorimétrie à balayage différentiel ;
dans laquelle la composition comprend un espacement d compris entre 10 nm et 32 nm, de préférence entre 15 nm et 30 nm, de préférence entre 17 nm et 29 nm, et de préférence encore entre 20 nm et 27 nm, tel que mesuré selon l'espacement d (espacement Lβ-basal) du procédé d'essai du réseau de gel de Lamella décrite dans le présent document.

2. Composition selon la revendication 1, dans laquelle le ou les alcools gras sont choisis dans le groupe constitué d'alcool cétylique, alcool stéarylique, alcool béhénylique, et combinaisons de ceux-ci.

3. Composition selon l'une quelconque des revendications précédentes, comprenant en outre de 0,25 % en poids à 6 % en poids d'un acide, de préférence de 0,4 % en poids à 5 % en poids d'un acide, plus préférablement de 0,5 % en poids à 4 % en poids d'un acide, et le plus préférablement de 0,6 % en poids à 3 % en poids d'un acide ; dans laquelle l'acide est choisi dans le groupe constitué d'acide I-glutamique, acide lactique, acide chlorhydrique, acide malique, acide succinique, acide acétique, acide fumarique, acide tartrique, acide citrique, chlorhydrate l-glutamique, acide maléique, et des mélanges de ceux-ci.

4. Composition selon l'une quelconque des revendications précédentes, comprenant en outre une teneur en réseau gélifié de 0,1 à 0,6 en mole, de préférence de 0,2 à 0,5 mole, et plus préférablement de 0,3 à 0,4 mole.

5. Composition selon l'une quelconque des revendications précédentes, comprenant une contrainte de cisaillement de 75 Pa à 575 Pa, de préférence de 100 Pa à 565 Pa, plus préférablement de 105 Pa à 550 Pa, et le plus préférablement de 125 Pa à 450 Pa, selon le procédé d'essai de contrainte de cisaillement décrit dans le présent document.

6. Composition d'après-shampooing selon l'une quelconque des revendications précédentes, dans laquelle la composition comprend moins de 0,5 % de chlorure de béhentrimonium, de chlorure de cétrimonium et/ou de stéaramidopropyl diméthylamine en poids de la composition.

7. Composition d'après-shampooing selon l'une quelconque des revendications précédentes, dans laquelle la composition comprend moins de 0,5 % en poids de la composition ou est exempte de sels d'ammonium quaternisé mono alkyle long.

8. Composition d'après-shampooing selon l'une quelconque des revendications précédentes, dans laquelle la composition comprend moins de 0,5 % d'un ingrédient en poids de la composition, dans laquelle l'ingrédient est choisi dans le groupe constitué de silicones, agents propulseurs, phtalates, colorants, sulfates, donneurs de formaldéhyde, et combinaisons de ceux-ci.

9. Composition de conditionnement selon l'une quelconque des revendications précédentes, dans laquelle la composition comprend un pH de 2,5 à 5, et de préférence de 3,5 à 4,5, tel que mesuré selon le procédé de test du pH, décrit dans le présent document.

10. Composition de conditionnement selon l'une quelconque des revendications précédentes, comprenant en outre un système de conservation comprenant :
a. de 0,1 % à 0,5 % de benzoate de sodium, de préférence de 0,2 % à 0,4 % de benzoate de sodium en poids de la composition ;
b. de 0,3 % en poids à 1,5 % en poids d'une seconde composition, de préférence de 0,32 % en poids à 1 % en poids d'une seconde composition, plus préférablement de 0,33 % en poids à 0,8 % en poids d'une seconde composition, et encore plus préférablement de 0,37 % en poids à 0,45 % en poids d'une seconde composition ; dans laquelle la seconde composition est choisie dans le groupe constitué d'un glycol, un ester glycérique et des combinaisons de ceux-ci ; dans laquelle le rapport de benzoate de sodium/seconde composition est de 1:4 à 1:1, de préférence de 1:3 à 1:1, plus préférablement de 1:2 à 1:1, et encore plus préférablement de 1:1,7 à 1:1.

11. Composition d'après-shampooing selon la revendication 10, dans laquelle la seconde composition comprend un glycol, dans laquelle le glycol étant choisi dans le groupe constitué de butylène glycol, pentylène glycol, hexylène glycol, 1,2-hexanediol, caprylyl glycol, décylène glycol, et mélanges de ceux-ci.

12. Composition d'après-shampooing selon la revendication 10 ou 11, dans laquelle la seconde composition comprend un ester de glycéryle, dans laquelle l'ester de glycéryle est choisi dans le groupe constitué de caprylate de glycéryle, caprate de glycéryle, undécylénate de glycéryle et mélanges de ceux-ci.

13. Composition d'après-shampooing selon la revendication 10, dans laquelle le niveau de microbes est indétectable après deux jours, tel que mesuré selon les procédés de test de sensibilité microbienne bactérienne et fongique.

14. Utilisation d'une composition d'après-shampooing selon l'une quelconque des revendications précédentes, pour fournir des avantages d'après shampooing satisfaisants en termes de sensation humide et de démêlage humide.
